# EUROPEAN PATENT APPLICATION

(11) **EP 4 756 824 A1**
(43) Date of publication of application: **10.06.2026**
(21) Application number: 25221290.7
(22) Date of filing: 05.12.2025
(51) Int. Cl.: G16H 20/40, A61B 17/04, A61B 34/20, A61B 34/00, A61B 34/30, A61B 34/32, G16H 40/63

(54) **ASSISTANCE ADVANCEMENT MULTI-SYSTEM INTERACTION**

(30) Priority: 06.12.2024 US 202418971606
(71) Applicant: Cilag GmbH International, 6300 Zug (CH)
(72) Inventor: SHELTON, IV,, Frederick E., Cincinnati, 45242 (US); HARRIS,, Jason L., Cincinnati, 45242 (US); COWPERTHWAIT,, Matthew David, Cincinnati, 45242 (US); ADAMS,, Shane R., Cincinnati, 45242 (US); ROSS,, Nicholas James, Cincinnati, 45242 (US)
(74) Representative: Carpmaels & Ransford LLP

(57) **Abstract**

A surgical system may include a processor configured to identify a surgical motion performed by a user. The surgical motion may be determined to be able to be automated by the surgical system. A plurality of assistance control options may be generated. An assistance control option may be applied from the plurality of assistance control options based on a selection made by the user. The surgical motion may be performed with the selected assistance control option. The surgical motion may be performed with the selected assistance control option after an indication from the user.

## Description

### CROSS-REFERENCE TO RELATED APPLICATIONS

The contents of each of the following, filed contemporaneously, are incorporated by reference herein:
- Attorney Docket No. END9638USNP1, entitled PROGRESSIVE ADVANCEMENT OF AUTOMATED LEVEL BASED ON LEARNED COMPLIMENTARY ASSISTANCE
- Attorney Docket No. END9638USNP2, entitled ADJUSTING AUTOMATED COOPERATIVE OPERATIONS BASED ON SITUATIONALLY DERIVED CONSTRAINTS,
- Attorney Docket No. END9638USNP4, entitled MONITORING AND IDENTIFYING SURGEON CONTROL AND SUGGESTING A TASK THAT MAY BE DONE AUTONOMOUSLY,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP6, entitled ADAPTIVE RETRACTION FORCE CONTROL,
- Attorney Docket No. END9638USNP7, entitled ADJUSTMENT OR DISPLAY OF OPTIONS OF POSITIONAL OR ORIENTATION IMPLICATIONS ON SURGICAL TOOL USAGE, and
- Attorney Docket No. END9638USNP8, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

A surgical system may include a processor configured to identify a surgical motion performed by a user. The surgical motion may be determined to be able to be automated by the surgical system. A plurality of assistance control options may be generated. An assistance control option may be applied from the plurality of assistance control options based on a selection made by the user. The surgical motion may be performed with the selected assistance control option. The surgical motion may be performed with the selected assistance control option after an indication from the user. The surgical motion may be a motion that has been performed (e.g., repeatedly performed) by the user, or a motion that is being performed (e.g., actively performed) by the user. The surgical motion may be a motion that is scheduled to be performed by the user (e.g., in an upcoming step of the surgical procedure).

In examples, the processor may be a first processor. The surgical system may further include a second processor configured to sense data of the patient. The data of the patient may be sent to the first processor.

In examples, each assistance control option of the plurality of assistance control options may include a unique level of automation. The surgical motion scheduled to be performed by the user may be identified before the user performs the surgical motion. The assistance control option may include a promotion. The assistance control option may include a demotion.

In examples, the assistance control option may meet criteria for advancement including an assessment of the surgical motion with the selected assistance control option. The surgical motion may be a minor surgical motion, for example, a noninvasive or minimally invasive surgical motion; a surgical motion at least a predetermined minimum distance away from one or more critical structures or vessels in the patient (such as one or more organs, veins, or arteries); a surgical motion which does not involve tissue dissection; a surgical motion with a footprint of motion within a predetermined size; or the like.

In examples, the surgical motion may be a suture performed by a user. The suture may be determined to be automated by the surgical system. A first assistance control option of low assistance and a second assistance control option of high assistance may be generated. The suture may be performed with the selected assistance control option under the surveillance of the user.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 shows an example computer-implemented surgical system for applying an assistance control option.
FIG. 7 shows an example computer-implemented surgical system for performing a suture with an assistance control option.
FIG. 8 shows an example flowchart for applying an assistance control option.

### DETAILED DESCRIPTION

A more detailed understanding may be had from the following description, given by way of example in conjunction with the accompanying drawings.

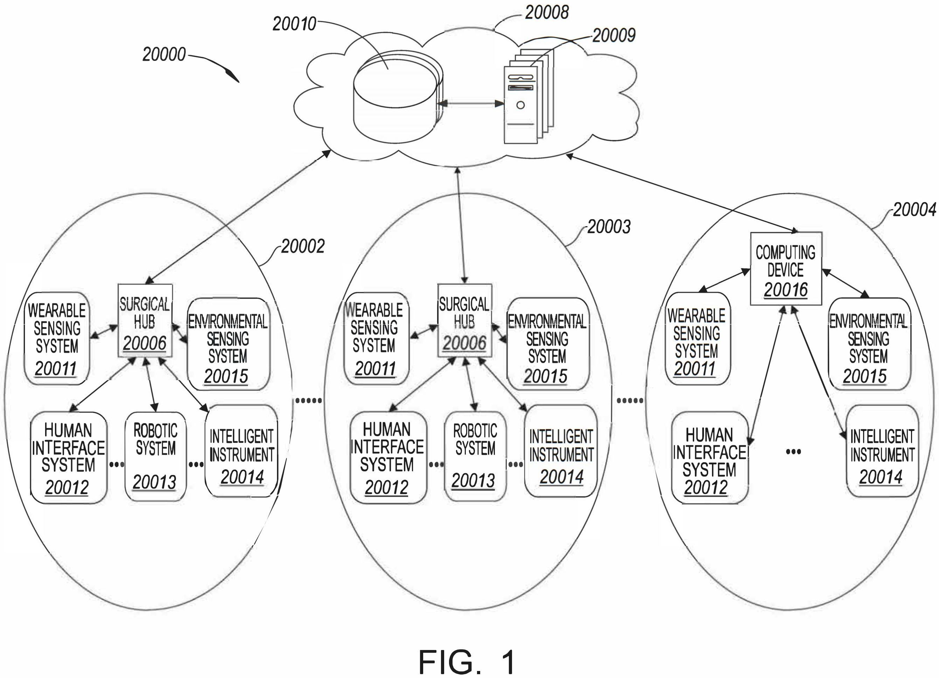

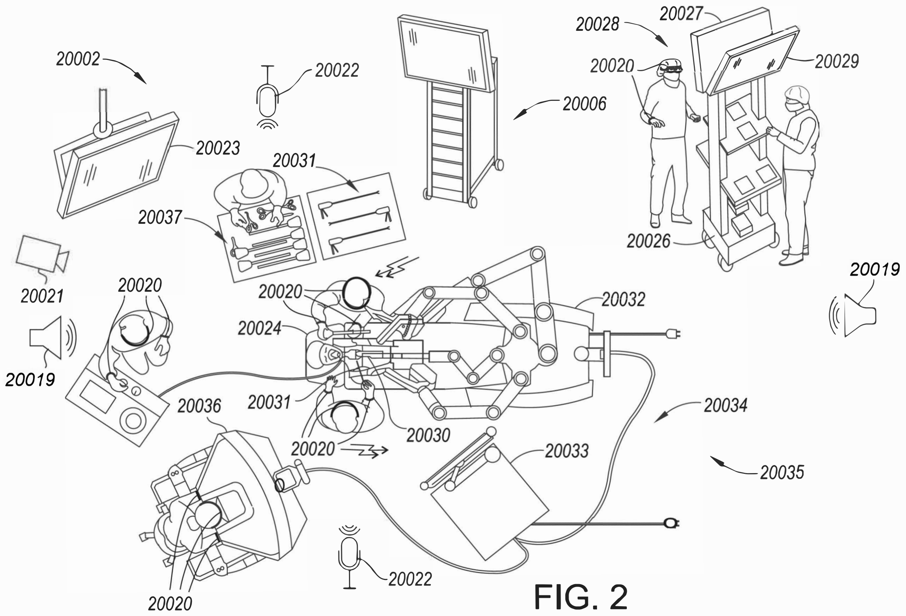

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngoneproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing. Sensed data may be used to determine an assistance control option. For example, a surgical system in accordance with embodiments of the present invention may comprise a processor configured to sense data of the patient and send the data of the patient to another processor, which can in turn apply an assistance control option from a plurality of assistance control options based on i) the sensed patient data, ii) a selection made by the user, or iii) a combination of i) and ii).

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

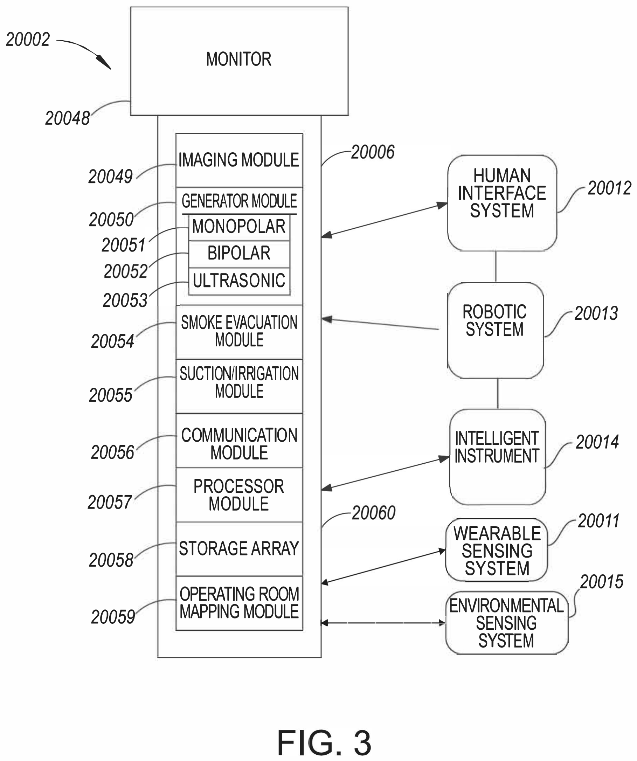

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

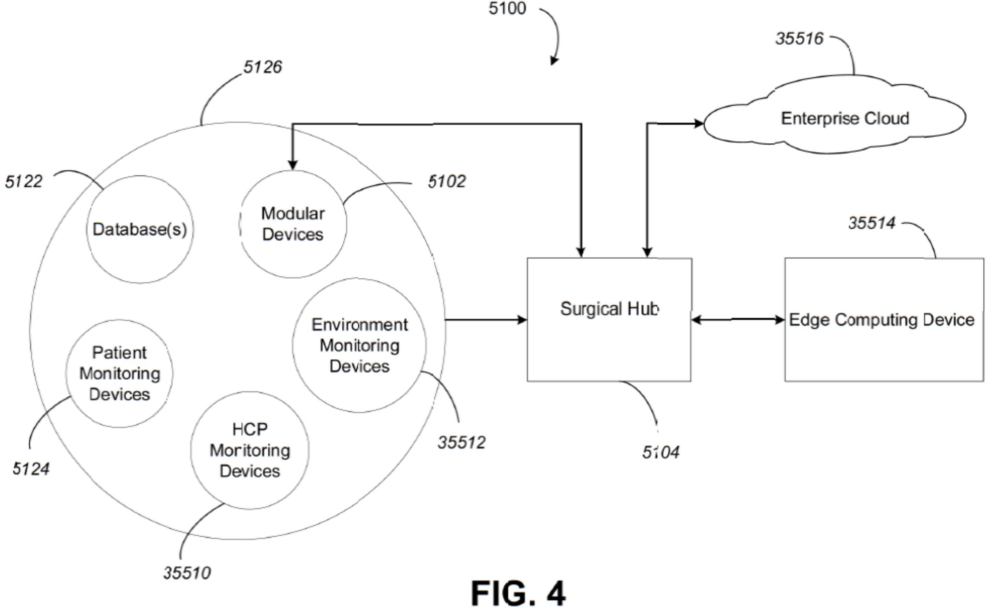

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an **RF** electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedureby-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

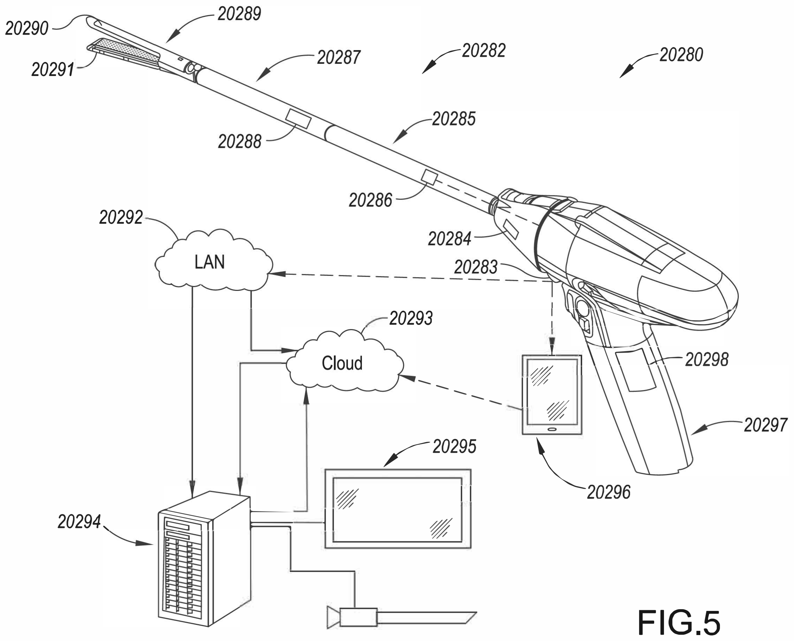

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

A surgical system may include a processor configured to identify a surgical motion performed by a user. The surgical motion may be determined to be able to be automated by the surgical system. A plurality of assistance control options may be generated. An assistance control option may be applied from the plurality of assistance control options based on a selection made by the user. The surgical motion may be performed with the selected assistance control option. The surgical motion may be performed with the selected assistance control option after an indication from the user. In general, instead of the processor/computer itself performing the surgical motion with the selected assistance control option, the processor/computer may simply cause the surgical motion to be performed with the selected assistance control option. For example, the processor/computer may cause a surgical system to perform (either a portion or the whole of) the surgical motion with the selected assistance control option. This surgical system may be, or may comprise, any suitable surgical system of those described herein. The surgical system may be a robotic surgical system, for example an autonomous robotic surgical system. In an exemplary combination, a first surgical system according to the present disclosure may comprise i) a processor, and ii) a second surgical system, wherein the processor is configured to identify a surgical motion performed by a user, determine that the surgical motion can be automated by the surgical system, generate a plurality of assistance control options, apply an assistance control option from the plurality of assistance control options based on a selection made by the user, and cause the second surgical system to perform the surgical motion with the selected assistance control option.

In examples, the processor may be a first processor. The surgical system may further include a second processor configured to sense data of the patient. The data of the patient may be sent to the first processor.

In examples, each assistance control option of the plurality of assistance control options may include a unique level of automation. The surgical motion scheduled to be performed by the user may be identified before the user performs the surgical motion. The assistance control option may include a promotion. The assistance control option may include a demotion.

In examples, the assistance control option may meet criteria for advancement including an assessment of the surgical motion with the selected assistance control option. The surgical motion may be a minor surgical motion.

In examples, the surgical motion may be a suture performed by a user. The suture may be determined to be automated by the surgical system. A first assistance control option of low assistance and a second assistance control option of high assistance may be generated. The suture may be performed with the selected assistance control option under the surveillance of the user.

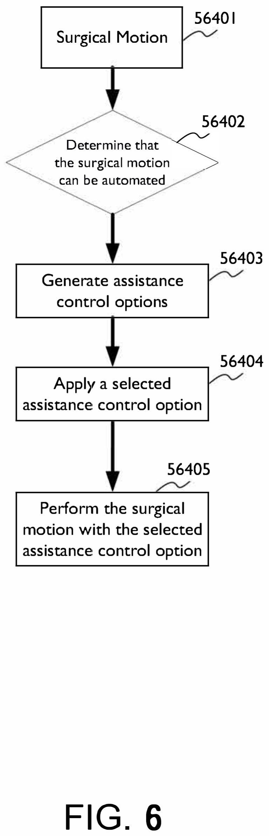

FIG. 6 shows an example computer-implemented surgical system applying an assistance control option. A surgical motion 56401 may be identified including but not limited to suturing, dissection, incising, grasping, holding, retracting, cutting, excision, cauterizing, coagulating, drilling, sawing, ligating, aspiration, suctioning, boring, inserting, implanting, etc. A smart system including a processor may capable of performing these motions. Depending on the motion, the surgeon may need to supervise the motion being performed to various degrees. The range of autonomy that may be provided varies vastly and may include as much as complete autonomy with supervision, to as little as virtual reality integration within the procedure through smart technology, for example.

The motion must be able to be automated to some degree for the smart system to generate assistance control options. The motion may be pre-identified in a number of ways including but not limited to the surgeon inputting into the system the procedure and/or motion to be performed. If the motion is determined to be able to be automated (step 56402), then the system may generate and/or provide a variety of assistance control options 56403 that the surgeon may choose from to apply to the motion by the system (step 56404). The motion may then be performed by the system with the selected assistance control option applied (step 56405).

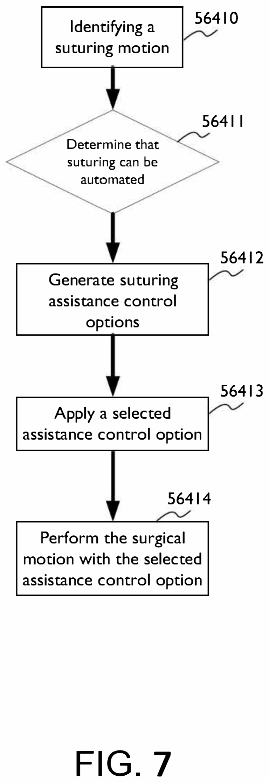

FIG. 7 shows an example computer-implemented surgical system for performing a suture with an applied assistance control option. The surgical motion may be defined as a suture 56410 on a patient in a surgical procedure. The suture may be determined to be able to be automated 56411 by the system. Autonomous suturing may involve the use of robotic systems and/or algorithms that perform sutures with minimal or no human intervention. Medical robotics may be integrated with computer vision, artificial intelligence (AI), and sensors, for example, to suture tissues. At 56412, suturing assistance control options are generated. At 56413, a selected assistance control option is applied. At 56414, the surgical motion is performed by the system with the selected assistance control option applied.

A robotic platform may be programmed to mimic the motions a surgeon would perform during suturing (e.g., needle driving, knot tying, and tissue handling). High-resolution cameras and 3D imaging systems may allow the robot to map the surgical site. Al-based image recognition algorithms may identify the tissue edges to be sutured. Depth perception (e.g., using stereo cameras and/or infrared) may ensure the robot can assess the correct entry and/or exit points for needle insertion. As the tissue is flexible and moves, the system must adjust in real time using tactile sensors to detect tissue tension, force feedback sensors to ensure the correct amount of pressure is applied, and/or soft-tissue modeling algorithms to predict tissue deformation and/or adjust motion.

The robot's arm may grip and/or position the needle accurately. Needle insertion may be through tissue at the planned entry point. Rotation of a wrist (e.g., a robotic end-effector) may follow a smooth curve through the tissue. The suture thread may be pulled through with minimal drag on the tissue. Automated knottying may be achieved by pre-programming specific knot types (e.g., surgeon's knot or square knot). The robot may loop the suture thread around itself and/or other tools to complete a secure knot.

If the needle deviates and/or if the suture tension is too loose or tight, the system may correct it using Al-based feedback loops. In examples, machine learning algorithms may enable continuous improvement over multiple operations. Path-planning algorithms may determine the optimal stitching route. Evenly spaced stitches may be placed along a wound. Adaptive algorithms may adjust stitch patterns based on tissue properties (e.g., elasticity or thickness).

The automation may be done at the (e.g., direct) direction of the healthcare professional (HCP) to accomplish (e.g., minor) adjustments and/or movements. Automated jobs (e.g., minor automated jobs) may be performed to surgically assist the surgeon (e.g., HCP). Smart systems (e.g., two) may cooperate and/or collaborate with each other and/or at least one utilizing sensed data provided by the other. HCP motions may be pre-identified. Techniques and/or behaviors may be automated by minor adjustments on a smart systems (e.g., one of). At the direction of the HCP, the automation may be utilized to accomplish the (e.g., pre-identified) motions.

Levels of automation (LoA) describe the extent to which a system or process may operate independently of human control. LoA frameworks may define how much a human is involved in decisionmaking and/or execution. The range of automation may extend from fully manual to fully autonomous systems. At a level 0 LoA, no automation is provided and it the process is manually controlled. Humans may perform all tasks without assistance from automated systems. In examples, the surgeon may make incisions and/or sutures manually. At a level 1 LoA, assisted automation may be provided. The system may offer basic assistance. The human operator may remain fully in control and/or may be responsible for decisions and actions. In examples, robotic instruments may provide tools for precise movement, but the surgeon may control every motion. At a level 2 LoA, partial automation may be provided. The system may perform some tasks independently. The human may monitor the system and take over when necessary. In examples, a surgical robot may follow pre-set paths for stitching while the surgeon oversees and/or adjusts. At a level 3 LoA, conditional automation may be provided. The system may operate autonomously under specific conditions but requires human intervention if it encounters a situation beyond its capabilities. In examples, a robotic system may autonomously perform suturing on soft tissue. The surgeon may take over if unexpected bleeding occurs. At a level 4 LoA, high automation may be provided. The system may perform all tasks within defined environments (e.g., like specific surgical settings or certain driving routes). Human intervention may be rarely required. It may not be able to handle every situation outside its designed scope. In examples, a robot may complete routine, low-risk surgeries (e.g., like simple tissue suturing) without human involvement, but a surgeon may be present to intervene if needed. At a level 5, full automation (e.g., autonomy) may be provided. The system may perform all tasks independently in any situation, with no need for human intervention. In examples, an autonomous robot may perform complex surgeries from start to finish, even making decisions in real time based on unexpected conditions.

Assist to the HCP by the system may be operated without an understudy. In examples, an assistant (e.g., Level 3) may automate minor moving or moderate displaying activities to minimize the cognitive burden on the HCP to accomplish the surgical steps of the procedure which may be complimentary to what the user is controlling.

The system may provide assisting operation of secondary, auxiliary, or related smart systems to take the cognitive burden off the surgeon (e.g., HCP) for the primary tasks that are being attended to. Automatic camera field-of-view maintenance may be provided. Augmenting imaging systems may activated and/or adjusted to provide complimentary views of the field being controlled by the surgeon (e.g., HCP). The attention of an HCP may be requested to off-screen interactions, key aspects, or items requiring attention based on criticality of the task being conducted (e.g., currently) by the HCP.

System homogenization may be interrelated. In examples, a stationary organ retraction may be homogenous. A separate analysis may be utilized to determine what may be automated. The user may be given the choice to select what to automate, and/or to determine the level or magnitude of the main automation. In examples, the system (e.g., assistant) may be asked to master a single skill within a surgical procedure before branching out into multiple skills or areas. In examples, the system may perform suturing numerous times and develop a basic level of competency in suturing (e.g., as well as the corresponding elements of arm positioning, etc.), before taking on additional skills even within the same promotional level.

The system may provide different levels of surgical support based on an aspect of (e.g., determined by) the HCP. In examples, for opening and closing a patient, the automated assistance may provide options for different (e.g., 'Co-Pilot') levels of automation. However, for laparoscopic dissection within the procedure, the robotic system may (e.g., only) provide levels of different (e.g., 'Assistant') levels of automation. For stapling actions, the system may (e.g., only) provide support (e.g., 'Understudy'). A second system may be integrated with the first system to acquire data of the patient. This data may be sent to the first system as a factor for determining the level of automation best fit for the procedure taking place.

The risk and/or complexity of the procedure may be taken into account to determine the level of automation offered to the HCP. A single skill, such as suturing, may encompass numerous facets. This may include straightforward suturing scenarios, and/or more complicated suturing scenarios based on tissue, anatomical access, and other constraints.

Identified and/or confirmed triggering may be utilized to activate an automated step after a user controlled step. Control of assistance advancement may be provided. Types of assistance advancement may include promotions, and demotions, for example. Promotions may include performance-based promotion. Advancement from one level of assistance to a different level of assistance may be monitored and/or controlled by the level of performance provided.

Performance feedback on system performance may be provided by direction surgeon input, indirect surgeon input, and/or absolute feedback or monitoring of metrics. Direct surgeon input (e.g., a survey, questionnaire, or rating system by the surgeon) may rank how the system performed during that surgery (e.g., as well as potentially the difficulty of the surgery). Indirect surgeon input may determine if the surgeon manually adjusted the system (e.g., repeatedly). Absolute feedback and/or monitoring of metrics may include a time to complete a surgery, the amount of bleeding, and/or other parameters that may be directly monitored.

Advancement from one level to the second level may include promotions, or demotions, for example. Promotion may include surgeon manual promotion (e.g. nepotism). Advancement may not be based directly on performance, but rather a surgeon judgement to advance the system to a determined level. In examples, the surgeon may require a higher level of assistance (e.g., if the surgery is relatively easy). As a result, the surgeon may choose to advance the system earlier than originally anticipated, which may allow the system to learn additional steps (e.g., at a faster rate).

Promotion may include time-based promotion. For time-based promotion, promotion may be handled if a minimum amount of time has been invested into a given procedure and/or set of surgical skills. In examples, a system may require a minimum of a certain number of (e.g., 300) hours doing a certain task to be promoted from one level to the next hierarchical level.

Promotion may include temporary and/or trial promotion to the subsequent level (e.g., a probationary period). In examples, the system may meet (e.g., all) the requirements to be promoted to the next level. Some skills may require 'on-the-job' training. The system may need to perform these skills to assess itself and/or improve. This may give the system the chance to perform them. However, it should see an improvement to some minimum level within a period of time, for example, 60 hours. If it fails to meet this additional improvement, it may be returned back to the prior level.

Advancement from one level to the second level may include demotion. Demotion may include surgeon-based demotion. Surgeon-based demotion may be temporary, permanent, be a time-based demotion, and/or performance-based demotion. For a temporary demotion, a surgeon may not feel comfortable and/or have confidence in the specific procedure they are performing using this automated assistance. As a result, the surgeon may temporarily demote the surgical assistance to a lower level. For a permanent demotion, the surgeon may permanently reduce the level of surgical assistance from the system based on their perception of how it has been performing. For time-based demotion, there may be a large gap between when the automated assistance was last utilized within a surgical procedure. As a result, there may be a risk in returning to the same level of autonomy. The system may be demoted to a lower level based on how long it has not been used. Performance-based demotion may include being demoted to a subsequent level if the system has already been promoted to a subsequent level, and based on how the system has been performing, it no longer meets the expected criteria for that level of automation. As a result, the system may be demoted back to a lower level of automation.

Criteria for advancement may include specific task assessments. The system may apply a hierarchy of assistance levels that are based on the application or use of specific tasks rather than at an overall system level. In examples, a system may be deemed (e.g., highly competent) and/or perform at a co-pilot level for more routine tasks that are performed such as assisting at suturing in the closing of a patient. Other tasks within the same surgery may not meet the same competency level.

Surgical Task Assessments may include suturing, dissection, etc. Functional Assessments may include mobilization of organs, opening and/or closing, etc. Boundary condition assessments may include skills the system may perform in normal operations but when brought to the limits of the system may prove more challenging and/or be outside the scope of skill assessments. Anatomical access may also be considered (e.g., if the robotic system able to reach certain regions). Factors that may be taken into account for anatomical access include but are not limited to arm length, joint access angles, tissue thickness out of indications, tissue may be too thick for the robotic stapler, challenging tissue conditions, necrotic tissue, etc.

Continual assessment of automation of the performance of the system may be provided. The continual assessment may include blinded assessment, surgeon assessment, self-assessment, etc. For surgeon assessment, the surgeon may provide feedback to the system on how it has performed certain tasks.

For self-assessment, assessment of the system, especially for more binary tasks that are more straightforward, may include defined success criteria. The system may continue to monitor it's performance for basic functionality which may be provided as control for advancement, promotion blocking, etc. The system may meet all the criteria to be promoted to the next level, but the surgeon or other surgical staff refuse to let the system be promoted.

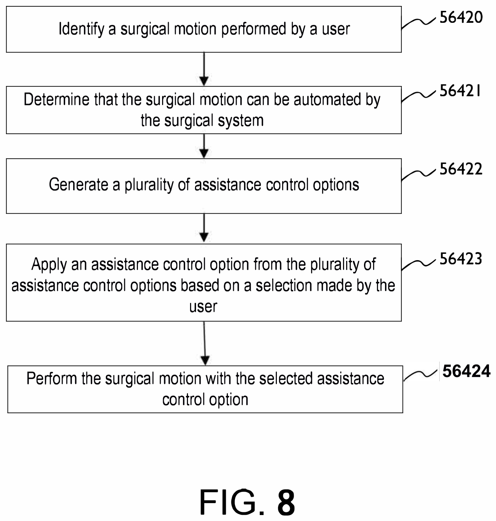

FIG. 8 shows an example flowchart for applying an assistance control option. At 56420, a surgical motion performed by a user may be identified. At 56421, the surgical motion may be determined to be able to be automated by the surgical system. At 56422, a plurality of assistance control options may be generated. At 56423, an assistance control option from the plurality of assistance control options may be applied based on a selection made by the user. At 56424, the surgical motion may be performed with the selected assistance control option. The following numbered list of examples forms part of the present disclosure.

Example 1. A surgical system comprising:
a processor configured to:
identify a surgical motion performed by a user;
determine that the surgical motion can be automated by the surgical system;
generate a plurality of assistance control options;
apply an assistance control option from the plurality of assistance control options based on a selection made by the user; and
perform the surgical motion with the selected assistance control option.

Example 2. The surgical system of example 1, wherein the processor is further configured to:
perform the surgical motion with the selected assistance control option after an indication from the user.

Example 3. The surgical system of any of examples 1-2, wherein the processor is a first processor, and wherein the surgical system further comprises:
a second processor is configured to:
sense data of the patient; and
send the data of the patient to the first processor.

Example 4. The surgical system of any of examples 1-3, wherein each assistance control option of the plurality of assistance control options comprises a unique level of automation.

Example 5. The surgical system of any of examples 1-4, wherein the surgical motion scheduled to be performed by the user is identified before the user performs the surgical motion.

Example 6. The surgical system of any of examples 1-5, wherein the assistance control option comprises a promotion.

Example 7. The surgical system of any of examples 1-6, wherein the assistance control option comprises a demotion.

Example 8. The surgical system of any of examples 1-7, wherein the assistance control option meets criteria for advancement comprising an assessment of the surgical motion with the selected assistance control option.

Example 9. The surgical system of any of examples 1-8, wherein the surgical motion is a minor surgical motion.

Example 10. The surgical system of any of examples 1-9, wherein:
the surgical motion is a suture performed by a user;
the suture is determined to be automated by the surgical system;
a first assistance control option of low assistance and a second assistance control option of high assistance are generated; and
the suture is performed with the selected assistance control option under the surveillance of the user.

Example 11. A surgical operating method comprising:
identifying a surgical motion performed by a user;
determining that the surgical motion can be automated by the surgical system;
generating a plurality of assistance control options;
applying an assistance control option from the plurality of assistance control options based on a selection made by the user; and
performing the surgical motion with the selected assistance control option.

Example 12. The surgical operating method of example 11, wherein the method further comprises:
performing the surgical motion with the selected assistance control option after an indication from the user.

Example 13. The surgical operating method of any of examples 11-12, wherein the method further comprises
sensing data of the patient; and
sending the data of the patient to the first processor.

Example 14. The surgical operating method of any of examples 11-13, wherein each assistance control option of the plurality of assistance control options comprises a unique level of automation.

Example 15. The surgical operating method of any of examples 11-14, wherein the surgical motion scheduled to be performed by the user is identified before the user performs the surgical motion.

Example 16. The surgical operating method of any of examples 11-15, wherein the assistance control option comprises a promotion.

Example 17. The surgical operating method of any of examples 11-16, wherein the assistance control option comprises a demotion.

Example 18. The surgical operating method of any of examples 11-17, wherein the assistance control option meets criteria for advancement comprising an assessment of the surgical motion with the selected assistance control option.

Example 19. The surgical operating method of any of examples 11-18, wherein the surgical motion is a minor surgical motion.

Example 20. The surgical operating method of any of examples 11-19, wherein:
the surgical motion is a suture performed by a user;
the suture is determined to be automated by the surgical operating method;
a first assistance control option of low assistance and a second assistance control option of high assistance are generated; and
the suture is performed with the selected assistance control option under the surveillance of the user.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A surgical system comprising:
a processor configured to:
identify a surgical motion performed by a user;
determine that the surgical motion can be automated by the surgical system;
generate a plurality of assistance control options;
apply an assistance control option from the plurality of assistance control options based on a selection made by the user; and
perform the surgical motion with the selected assistance control option.

2. The surgical system of claim 1, wherein the processor is further configured to:
perform the surgical motion with the selected assistance control option after an indication from the user.

3. The surgical system of claim 1 or claim 2, wherein the processor is a first processor, and wherein the surgical system further comprises:
a second processor is configured to:
sense data of the patient; and
send the data of the patient to the first processor.

4. A computer-readable medium comprising instructions which, when executed by a computer, cause the computer to:
identify a surgical motion performed by a user;
determine that the surgical motion can be automated by the surgical system;
generate a plurality of assistance control options;
apply an assistance control option from the plurality of assistance control options based on a selection made by the user; and
perform the surgical motion with the selected assistance control option.

5. The computer-readable medium of claim 4, wherein the instructions further cause the computer to perform the surgical motion with the selected assistance control option after an indication from the user.

6. The computer-readable medium of claim 4 or claim 5, wherein the instructions further cause the computer to receive sensed patient data from a processor or surgical sensing system.

7. The surgical system or computer-readable medium of any preceding claim, wherein each assistance control option of the plurality of assistance control options comprises a unique level of automation.

8. The surgical system or computer-readable medium of any preceding claim, wherein the surgical motion scheduled to be performed by the user is identified before the user performs the surgical motion.

9. The surgical system or computer-readable medium of any preceding claim, wherein the assistance control option comprises a promotion.

10. The surgical system or computer-readable medium of any preceding claim, wherein the assistance control option comprises a demotion.

11. The surgical system or computer-readable medium of any preceding claim, wherein the assistance control option meets criteria for advancement comprising an assessment of the surgical motion with the selected assistance control option.

12. The surgical system or computer-readable medium of any preceding claim, wherein the surgical motion is a minor surgical motion.

13. The surgical system or computer-readable medium of any preceding claim, wherein:
the surgical motion is a suture performed by a user;
the suture is determined to be automated by the surgical system;
a first assistance control option of low assistance and a second assistance control option of high assistance are generated; and
the suture is performed with the selected assistance control option under the surveillance of the user.
